# EUROPEAN PATENT SPECIFICATION

(11) **EP 2 381 967 B1**
(45) Date of publication and mention of the grant of the patent: **18.01.2017**
(21) Application number: 09837135.4
(22) Date of filing: 29.12.2009
(51) Int. Cl.: A61K 51/04, A61M 36/14, C07D 213/16

(54) **SYNTHESIS OF 18F-RADIOLABELED STYRYLPYRIDINES FROM TOSYLATE PRECURSORS AND STABLE PHARMACEUTICAL COMPOSITIONS THEREOF**
SYNTHESE VON 18F-RADIOAKTIV MARKIERTEN STRYRYLPYRIDINEN AUS TOSYLAT-VORSTUFEN UND STABILE PHARMAZEUTISCHE ZUSAMMENSETZUNGEN DARAUS
SYNTHÈSE DE STYRYLPYRIDINES RADIOMARQUÉES PAR 18F À PARTIR DE PRÉCURSEURS DE TOSYLATE ET LEURS COMPOSITIONS PHARMACEUTIQUES STABLES

(30) Priority: 31.12.2008 US 141885 P
(43) Date of publication of application: 02.11.2011
(73) Proprietor: Avid Radiopharmaceuticals, Inc., Philadelphia, PA 19104 (US)
(72) Inventor: BENEDUM, Tyler, Philadelphia Pennsylvania 19129 (US); GOLDING, Geoff, Philadelphia Pennsylvania 19145 (US); LIM, Nathaniel, Philadelphia Pennsylvania 19128 (US); ZHANG, Wei, Havertown Pennsylvania 19083 (US)
(74) Representative: O'Connor, David
(86) International application number: PCT/US2009/069741
(87) International publication number: WO 2010/078370

(56) References cited:
- US-A1- 2007 281 299
- US-A1- 2008 038 195
- SCOTT P J H ET AL: "Studies into radiolytic decomposition of fluorine-18 labeled radiopharmaceuticals for positron emission tomography", APPLIED RADIATION AND ISOTOPES, ELSEVIER, OXFORD, GB, vol. 67, no. 1, 7 September 2008 (2008-09-07), pages 88-94, XP025686472, ISSN: 0969-8043, DOI: 10.1016/J.APRADISO.2008.08.015 [retrieved on 2008-09-07]
- SOUZA ET AL.: 'Longitudinal noninvasive PET-based Beta cell mass estimates in a spontaneous diabetes rat model.' JOURNAL OF CLINICAL INVESTIGATION vol. 116, June 2006, pages 1506 - 1513
- ZHANG ET AL.: 'F-18 Stilbenes as PET Imaging Agents for Detecting Beta-Amyloid Plaques in the Brain.' JOURNAL OF MEDICINAL CHEMISTRY vol. 48, 2005, pages 5980 - 5988

## Description

### FIELD OF INVENTION

The present invention relates generally to synthesis methods for ¹⁸F-radiolabeled brain imaging agents and more specifically to methods of synthesizing ¹⁸F-radiolabeled styrylpyridine and its tosylate precursor and to stable pharmaceutical compositions comprising ¹⁸F-radio labeled brain imaging agents.

### DESCRIPTION OF RELATED ART

Alzheimer's Disease (AD) is a progressive neurodegenerative disorder characterized by cognitive decline, irreversible memory loss, disorientation, and language impairment. AD affects 10% of the population aged greater than 65 and at least 50% of the population aged greater than 85 years. AD has been reported in patients as young as 40-50 years of age, but because the presence of the disease is difficult to detect without histopathological examination of brain tissue, the time of onset in living subjects is unknown.

Currently, the only means of definitively diagnosing AD is through examination of brain tissue, typically performed at postmortem autopsy. During the autopsy, medical examiners inspect the brain tissue for excess neuritic plaques (NPs) composed of amyloid-β peptide deposits and neurofibrillary tangles (NFTs) formed of filaments of highly phosphorylated tau proteins, as these features distinguish the pathogenesis of AD. The amyloid deposits are formed by aggregation of amyloid peptides, followed by further combination with other aggregates and/or amyloid peptides. The fibrillar aggregates of amyloid peptides, Aβ1-40 and Aβ1-42, are the major peptide metabolites derived from amyloid precursor protein that are found in NPs and cerebrovascular amyloid deposits in AD patients.

Parkinson's Disease (PD) is a progressive neurodegenerative disease characterized by resting tremors, bradykinesia, muscular rigidity, and postural instability. PD typically develops after the age of 60, though 15% of diagnosed patients are under the age of 50. Family history of PD is an etiological factor for 5-10% of patients diagnosed with the disease, yet only 1% of cases have been shown to be clearly familial. It is estimated that 1.5 million Americans are currently living with PD.

Dementia with Lewy Bodies (DLB) is a progressive brain disease having symptoms that fluctuate between various degrees of manifestation. These symptoms include progressive dementia, Parkinsonian movement difficulties, hallucinations, and increased sensitivity to neuroleptic drugs. As with AD, advanced age is considered to be the greatest risk factor for DLB, with average onset usually between the ages of 50-85. Further, 20% of all dementia cases are caused by DLB and over 50% of PD patients develop "Parkinson's Disease Dementia" (PDD), a type of DLB. It is possible for DLB to occur alone, or in conjunction with other brain abnormalities, including those involved in AD and PD, as mentioned above. At present, a conclusive diagnosis of DLB is only possible after postmortem autopsy.

PD and DLB share an etiology of dopamine deficiency that is correlated with the death of dopaminergic neurons in the substantia nigra. The cause of dopaminergic neuronal death in PD is uncertain, although it appears that aggregates of α-synuclein in the brain may be associated with dopaminergic neuronal losses in the striatum. It is also recognized that in DLB, abnormal protein deposits containing α-synuclein, referred to as "Lewy bodies", are the cause of the death of dopaminergic neurons. Lewy bodies occur mostly in the substantia nigra and locus ceruleus sections of the brain stem, and also in the subcortical and cortical regions of the brain. Because of this particular localization in the brain, Lewy bodies may interfere with the production of acetylcholine, causing disruption of perception and thought process and impacting behavior. Lewy bodies are considered to be a type of neuritic plaque (NP) because they are comprised of aggregates of α-synuclein protein deposits.

The etiology of neuro degeneration can also involve a mixture of pathologies including a component of microvascular, or perfusion, deficits in the brain. For example, a disorder commonly referred to as "mixed dementia" often comprises both perfusion deficits and amyloid plaque pathology. The term "mixed dementia" possesses various meanings, but the term is commonly used to refer to the coexistence of AD and vascular dementia (VaD), in particular where the VaD is caused by numerous micro-thrombi in the vascular system of the brain. Though little is currently known about the true prevalence of mixed dementia, this form of neurodegeneration is clinically important because the combination of AD and VaD may have a greater impact on the brain than either condition independently. Mixed dementia is traditionally very difficult to diagnose. Symptoms are similar to those of AD or VaD or a combination of the two.

The occurrence of amyloid deposits in the brain may be characteristic of numerous other conditions including, but not limited to, Mediterranean fever, Muckle-Wells syndrome, idiopathetic myeloma, amyloid polyneuropathy, amyloid cardiomyopathy, systemic neuritic amyloidosis, amyloid polyneuropathy, hereditary cerebral hemorrhage with amyloidosis, Down's syndrome, Scrapie, Creutzfeldt-Jacob disease, Kuru, Gerstamnn-Straussler-Scheinker syndrome, medullary carcinoma of the thyroid, isolated atrial amyloid, β₂-microglobulin amyloid in dialysis patients, inclusion body myositis, β₂-amyloid deposits in muscle wasting disease, and islets of Langerhans diabetes Type II insulinoma.

Because of the role of the presence of neuritic plaques (NPs) in the diagnosis of neurodegenerative disease, there has been interest in developing radiolabeled ligands that bind to and allow imaging of such abnormalities using current methodologies. Some common imaging agents include (¹¹C]P1B, [¹¹C] 4-N-methylamino-4'-hydroxy-stilbene (SB-13), [¹⁸F]FDDNP and [¹²³I]IMPY.

[¹⁸F]AV-45 ("¹⁸F-AV-45"), ((E)-4-(2-(6-(2-(2-(2-[¹⁸ F]fluoroethoxy)ethoxy)ethoxy) pyridine-3-yl)vinyl)-N-methylbenzenamine, is a radiopharmaceutical for Positron Emission Tomography (PET) imaging of amyloid aggregates in the brain (see, for example, Choi, SR, et al., *J Nucl Med,* 50(1 1), 1887- 1894, 2009). ¹⁸F-AV-45 contains the F- 18 radioisotope which, due to its 110 minute radioactive decay half life, may be manufactured in a centralized location and shipped within a 4 to 8 hour radius to imaging centers that perform the PET brain imaging. This production and distribution scheme for ¹⁸F-AV-45, and for other F-18 radiolabeled amyloid imaging compounds, requires that the radiopharmaceutical be produced on the same day on which the PET scan is performed because of the radioactive decay of the F-18 isotope. Therefore, several requirements result: first, the radioactive ¹⁸F-AV-45 must be produced in a short period of time from a stable intermediate compound (referred to as the "precursor"); second, the ¹⁸F-AV-45 radiopharmaceutical must be produced in reasonable (e.g., >10%) radiochemical yield starting from the precursor reacting with F-18 fluoride ion; and third, the ¹⁸F-AV-45 must be in a solution medium which provides adequate solubility of both the radiopharmaceutical as well as its non-radioactive counterpart (¹⁹F-AV-45, which is always present at some low level due to stable F-19 in the manufacturing environment), as well as stabilization properties to inhibit the radio lytic breakdown of the compound.

US 2008/038195 discloses also the ¹⁸F-AV-45 radiopharmaceutical.

Ethanol is generally recognized as a suitable aid for solubilization of lipophilic drugs, including radiopharmaceutical drugs (see, for example, Lemaire C. et al., J Label Compd and Radiopharm, 42, 63-75, 1999). Ascorbic acid or ascorbate salts have been utilized previously as aids in inhibiting radio lysis of radiopharmaceuticals (see, for example, Tofe AJ, et al., J. Nucl Med, 17, 820-825, 1976; Knapp FF, et al., Anticancer Res 17, 1783-1795, 1997; Liu S, et al., Bioconj Chem, 14, 1052-1056, 2003), including F-18 radiopharmceuticals, see, for example, Firnau G, et al., J Nucl Med, 25, 1228-1233, 1984). However, the use of ascorbate salts in an aqueous-ethanol solution as a preferred medium for both solubilization and stabilization of an F-18 brain imaging radiopharmaceutical, such as ¹⁸F-AV-45, is new.

The use of a precursor containing a tosylate group for reaction with F-18 fluoride in the production of F-18-containing radiopharmaceuticals has been previously described (see, for example, Zhang W, et al., Nucl Med Biol 34, 89-97, 2007). However, an efficient synthetic process for producing larger (i.e., >10gm) quantities of the tosylate precursor (referred to as "AV-105" herein) has not been described previously.

### SUMMARY OF THE INVENTION

The present invention is directed to a method for producing a radiopharmaceutical composition for positron emission tomography (PET) imaging of neurodegenerative diseases of the brain comprising:
an effective amount of ((E)-4-(2-(6-(2-(2-(2-[¹⁸F]fluoroethoxy)ethoxy)ethoxy)pyridin-3-yl)vinyl)-N-methylbenzenamine); 10.0% (v/v) of ethyl alcohol; and 0.5 % (w/v) of sodium ascorbate, in 0.9% (w/v) aqueous sodium chloride,
wherein the method comprises the steps of: preparing a mono Boc-protected vinylaniline compound; converting the vinylaniline compound to a methyl, t-butyl carbamate derivative;
reacting 2-halo 5-iodopyridine with triethyleneglycol; reacting the methyl, t-butyl carbamate derivative with the resultant compound of the step of reacting 2-halo 5-iodopyridine with triethyleneglycol to produce (E)-tert-Butyl 4-(2-(6-(2-(2-(2-hydroxyethoxy)ethoxy)ethoxy)pyridin-3-yl)vinyl)phenyl(methyl)carbamate; and
reacting the (E)-tert- Butyl 4-(2-(6-(2-(2-(2-hydroxyethoxy)ethoxy)ethoxy)pyridin-3-yl)vinyl)phenyl(methyl)carbamate with tosyl chloride to form (E)-2-(2-(2-(5-(4-(tert-butoxycarbonyl(methyl)amino)styryl)pyridin-2-yloxy)ethoxy)ethoxy)ethyl 4-methylbenzene sulfonate; reacting (E)-2-(2-(2-(5-(4-(tert-butoxycarbonyl(methyl)amino)styryl)pyridin-2-yloxy)ethoxy)ethoxy)ethyl
4-methylbenzene sulfonate and an [18F]-fluoride ion in dimethylsulfoxide (DMSO) solution or high-boiling point aprotic solvent to produce (E)-4-(2-(6-(2-(2-(2-[¹⁸F]fluoroethoxy)ethoxy)ethoxy)pyridin-3-yl)vinyl)-N-methylbenzenamine);
isolating the (*E*)-4-(2-(6-(2-(2-(2-[¹⁸F]fluoroethoxy)ethoxy)ethoxy)pyridin-3-yl)vinyl)-N-methylbenzenamine);
purifying the (*E*)-4-(2-(6-(2-(2-(2-[¹⁸F]fluoroethoxy)ethoxy)ethoxy)pyridin-3-yl)vinyl)-N-methylbenzenamine); and
formulating the (*E*)-4-(2-(6-(2-(2-(2-[¹⁸F]fluoroethoxy)ethoxy)ethoxy)pyridin-3-yl)vinyl)-N-methylbenzenamine) in an 0.9% (w/v) aqueous sodium chloride-ethyl alcohol solution comprising ethyl alcohol at 10.0% (v/v) of the total composition and sodium ascorbate at 0.5 % (w/v) of the total composition.

In another aspect of the invention, there is provided a compound of the formula

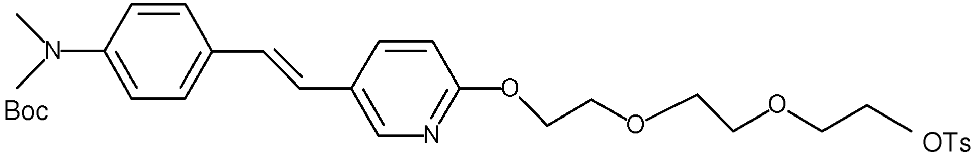

The radiopharmaceutical composition produced according to the method of the invention can be used in the diagnosis of neurodegenerative disease such as, for example, dementia, cognitive impairment, Alzheimer's Disease (AD), Parkinson's Disease (PD), Dementia with Lewy Bodies (DLB), Vascular Dementia (VaD) and combinations thereof.
The method according to the invention is suitable for producing an ¹⁸F-radiolabeIed composition capable of binding to β-amyloid and comprises the steps of synthesizing a tosylate precursor; performing nucleophilic ¹⁸F fluorination of the tosylate precursor in a dimethylsulfoxide (DMSO) solution to provide an ¹⁸F radiopharmaceutical; and formulating the ¹⁸F radiopharmaceutical in an aqueous-ethyl alcohol solution containing ascorbic acid or a salt thereof; wherein the ethyl alcohol is present at a concentration of 10% (v/v) and the concentration of the ascorbic acid or salt thereof is 5% (w/v) in the final ¹⁸F-radiolabeled pharmaceutical composition.

The method of the invention includes steps for producing the tosylate precursor of ¹⁸F-AV-45,(E)-2-(2-(2-(5-(4-(tert-butoxycarbonyl(methyl)amino)styryl)pyridin-2-yloxy)ethoxy) ethoxy)ethyl 4-methylbenzenesulfonate ("AV- 105"). These steps include (i) preparing a mono Boc-protected vinylaniline compound; (ii) converting the vinylaniline compound to a methyl, t-butyl carbamate derivative; (iii) reacting 2-halo 5-iodopyridine with triethyleneglycol; (iv) reacting the methyl, t-butyl carbamate derivative of step (ii) with the resultant compound of step (iii) to produce (E)-tert-Butyl 4-(2-(6-(2-(2-(2-hydroxyethoxy)ethoxy)ethoxy)pyridin-3-yl)vinyl)phenyl(methyl)carbamate; and (v) reacting the (E)-tert-Butyl 4-(2-(6-(2-(2-(2-hydroxyethoxy)ethoxy)ethoxy)pyridin-3-yl)vinyl)phenyl(methyl)carbamate with tosyl chloride to form AV-105.

The method of the invention also includes steps for producing a radiopharmaceutical composition including reacting the AV-105 tosylate precursor with an ¹⁸F-fluoride ion in dimethylsulfoxide (DMSO) solution or other high-boiling point aprotic solvent to produce ((E)-4-(2-(6-(2-(2-(2-[¹⁸F]fluoroethoxy)ethoxy)ethoxy)pyridin-3-yl)vinyl)-N-methylbenzenamine) (¹⁸F-AV-45); isolating the ¹⁸F-AV-45; and purifying the ¹⁸F-AV-45. The method of the invention further includes the step of formulating the ¹⁸F-AV-45 in an 0.9% (w/v) aqueous sodium chloride-ethyl alcohol solution comprising ethyl alcohol at 10% (v/v) of the total composition and sodium ascorbate at 5% (w/v) of the total composition.

### BRIEF DESCRIPTION OF THE FIGURES

For a better understanding of the disclosure, reference should be made to the following figures, in which:
FIG. 1 shows a schematic of the synthesis of tosylate precursor AV-105 and the synthesis of non-radioactive ¹⁹F-AV-45 from p-toluenesulfonate;
FIG. 2 shows an alternative synthesis method for tosylate precursor AV-105 according to the invention; and
FIG. 3 depicts a radiosynthesis method of one aspect of the invention using a tosylate precursor to produce β-amyloid binding radiopharmaceutical ¹⁸F-AV-45.

### DESCRIPTION OF ILLUSTRATIVE EMBODIMENTS

As used herein, the singular forms "a", "an" and "the" include plural reference unless the context clearly dictates otherwise.

As used herein, the term "about" means plus or minus 10% of the numerical value of the number unless otherwise described.

"Administering" when used in conjunction with an diagnostic agent, such as, for example, a radiopharmaceutical, means to administer directly into or onto a target tissue or to administer the radiopharmaceutical systemically to a patient whereby the diagnostic agent is used to image the tissue or a pathology associated with the tissue to which it is targeted. "Administering" a composition may be accomplished by injection, infusion, or by either method in combination with other known techniques.

The terms "comprise", "contain", "have" and "include" and their conjugates, as used herein, mean "including, but not necessarily limited to."

An "effective amount" or "therapeutically effective amount", as used herein, refers to the amount of positron-emitting radiopharmaceutical that results in a sufficient gamma radiation signal to adequately image the biological target of interest in the brain of an individual with a suspected neurodegenerative disease.

The term "end-of synthesis" or "EOS", as used herein, means end-of-radiosynthesis. This is the timepoint at which the radiosynthesis process, including any purification steps required for isolation of the radiopharmaceutical, are completed.

The term "high-boiling point aprotic solvent", as used herein, is defined as an aprotic solvent having a boiling point of at least about 140 °C at one (1) atm.

The terms "pathology" or "pathologic", as used herein, refer to an altered biological process that may be, for example, associated with aberrant production of proteins, peptides, RNA, and other substances associated with a disease process.

As used herein, the terms "patient" and "subject" refer to any living organism to which the compounds described herein are administered and whose brain activity is to be measured in conjunction with performing the analysis methods of the invention. Patients and/or subjects may include, but are not limited to, any non-human mammal, primate or human. Such patients and/or subjects may or may not be exhibiting signs, symptoms or pathology of one or more particular diseased states.

The term "target" when used in conjunction with a diagnostic agent, such as the radiopharmaceutical of the present invention, refers to tissue or other material associated with pathology to which localization of the radiopharmaceutical or diagnostic agent is desired. Targets may include, but are not limited to, diseased cells, pathogens, infectious material or other undesirable material in a patient, such as abnormal proteins, peptides, RNA or DNA or normally-expressed receptors that are altered in a disease process.

Generally speaking, as used herein, the term "tissue" refers to any aggregation of similarly specialized cells that are united in the performance of a particular function.

Various embodiments of the invention provide a method for the synthesis of an ¹⁸F-radiolabeled imaging agent from a tosylate precursor and a pharmaceutical composition of a stable drug product comprising such ¹⁸F-radio labeled imaging agent.

There is disclosed a radiopharmaceutical composition comprising an effective amount of an ¹⁸F-radiolabeled compound, such as an ¹⁸F-radiolabeled styrylpyridine or an ¹⁸F-Iabeled dihydrotetrabenazine (DTBZ) derivative, in a solution containing 10% (v/v) of ethyl alcohol, and 5 % (w/v) sodium ascorbate. There is also disclosed a radiopharmaceutical composition comprising an effective amount of an ¹⁸F-radiolabeled compound in a solution for intravenous injection containing 10% (v/v) of ethyl alcohol, and at least 0.5% (w/v) sodium ascorbate with a pH of between approximately 4.5 and 8.0. This radiopharmaceutical composition is a clear solution, without insoluble matter, which is stable for at least 6 hours following production at concentrations up to 100 mCi/mL (37 to 3700 MBq/mL) or more of the F-18 radiopharmaceutical.

The parenteral radiopharmaceutical compositions may contain a F-18 radio labeled compound which is capable of binding to a pathologic target in a brain of a patient such as, for example, an abnormal concentration of a native or pathologically-altered protein, peptide or oligonucleotide, β-amyloid, α-synuclein, or to a normally-expressed endogenous target that is impaired in the presence of certain degenerative disorders such as the vesicular monoamine transporter (VMAT2) in Parkinson's Disease (PD), Dementia with Lewy Bodies (DLB), or diabetes.

There is also disclosed radiopharmaceutical compositions having characteristics including: a high affinity and selectivity for the target, low molecular weight (<400 g/mol), medium lipophilicty (log P in the range of 1 -3) to ensure high initial brain uptake with rapid clearance, functional groups in the molecule for introduction of positron emitting radionuclides such as, for example, ¹¹C or ¹⁸F, high stability in the brain as well as no brain uptake of peripherally generated metabolites of the tracer compound, and high availability of the tracer compound for clinical centers.

Use of the ¹⁸F-labeled imaging agents of the invention provides many logistic advantages due to the relatively large half life of ¹⁸F (t_{1/2}= 110 min) as compared to other radioisotopes such as, for example, ¹¹C (t_{1/2}= 20 min). The relatively longer half-life of ¹⁸F is advantageous in that more time is provided for clearance of non-specifically bound tracer with less loss of signal strength due to radioactive decay.

The ¹⁸F-radiolabeled compound in the radiopharmaceutical composition comprises an ¹⁸F-radiolabeled compound, which is capable of binding to amyloid aggregates for use in positron emission tomography (PET) imaging of the brain. In various aspects, the ¹⁸F-radiolabeled compound is ((E)-4-(2-( 6-(2-(2-(2-[¹⁸F]fluoroethoxy)ethoxy)ethoxy)pyridin-3-yl)vinyl)-N-methylben/enamine) ("¹⁸F-AV-45"). In some embodiments, the ¹⁸F-AV-45 is produced from a tosylate precursor.

There is disclosed a radiopharmaceutical composition is provided comprising an effective amount of an ¹⁸F-radiolabeled compound, 10% (v/v) of ethyl alcohol, and 0.5% (w/v) ascorbate salt (e.g., sodium ascorbate), wherein the radiopharmaceutical composition retains a radiochemical purity of the desired radiopharmaceutical of >90% over a period of at least 4 hours after radiosynthesis, and more preferably over a period of up to 8 hours after radiosynthesis.

The pH of the composition can range from about 4.5 to about 8.0. In some embodiments, the purity of the radiopharmaceutical is at least 90% when measured at least about 2 hours post end-of-synthesis (EOS). In preferred embodiments, the purity of the radiopharmaceutical composition is at least 90% when measured at least about 4 hours post EOS.

The method according to the invention is suitable for producing an ¹⁸F-radiolabeled composition capable of binding to β-amyloid including synthesizing a tosylate precursor; performing nucleophilic ¹⁸F fluorination of the tosylate precursor; formulating the ¹⁸F fluorinated tosylate precursor in an aqueous solution; adding ethyl alcohol to achieve a concentration of about 10% (v/v) in the final ¹⁸F-radiolabeled styrylpyridine composition; and adding sodium ascorbate to achieve a concentration of 0.5% (v/v) in the final ¹⁸F-radiolabeled styrylpyridine composition.

The method according to the invention comprises steps for producing the tosylate precursor of ¹⁸F-AV-45, (E)-2-(2-(2-(5-(4-(tert-butoxycarbonyl(methyl)amino)styryl)pyridin-2-yloxy)ethoxy) elhoxy)ethyl 4-methylbenzenesulfonate ("AV- 105"), is provided. These steps include (i) preparing a mono Boc-protected vinylaniline compound; (ii) converting the vinylaniline compound from (i) to an N-methyl, t-butyl carbamate (i.e., N-methyl, N-Boc) derivative; (iii) reacting 2-halo 5-iodopyridine (in some embodiments halo = chloro or bromo); with triethyleneglycol (iv) reacting the N-methyl, N-Boc derivative of step (ii) with the resultant compound of step (iii) to produce (E)-tert-Butyl 4-(2-(6-(2-(2-(2-hydroxyethoxy)ethoxy)ethoxy)pyridin-3-yl)vinyl)phenyl(methyl) carbamate; and (v) reacting the (E)-tert-Butyl 4-(2-(6-(2-(2-(2-hydroxyethoxy)ethoxy)elhoxy)pyridin-3-yl)vinyl)phenyl(methyl) carbamate with tosyl chloride to form AV-105.

The method according to the invention also includes steps for producing a radiopharmaceutical composition including reacting the AV-105 tosylate precursor with an ¹⁸F-fluoride ion in DMSO or other high boiling aprotic solvent to produce ((E)-4-(2-(6-(2-(2-(2-[ ¹⁸F]fluoroethoxy)ethoxy)ethoxy)pyridin-3-yl)vinyl)-N-methylbenzenamine) ("¹⁸F-AV-45"); isolating the ¹⁸F-AV-45; and purifying the ¹⁸F-AV-45. The method of producing the radiopharmaceutical composition further includes the step of formulating the ¹⁸F-AV-45 in an 0.9% (w/v) aqueous sodium chloride-ethyl alcohol solution comprising ethyl alcohol at 10% v/v) of the total composition and sodium ascorbate at 5% (w/v) of the total composition.

There is disclosed a radiopharmaceutical composition comprising an effective amount of an ¹⁸F-radiolabeled compound, at least about 1.0% (v/v) of ethyl alcohol, and at least about 0.1% (w/v) sodium ascorbate, wherein the radiopharmaceutical composition decomposes at a rate measured in vitro that substantially corresponds to the following: (a) from about 0.01 to about 5% of the total decomposition after about 2 hours of measurement; (b) from about 0.01 to about 10% of the total decomposition after about 10 hours of measurement; (c) from about 0.01 to about 20% of the total decomposition after about 15 hours of measurement; and (d) not more than about 25% of the total decomposition after about 24 hours of measurement.

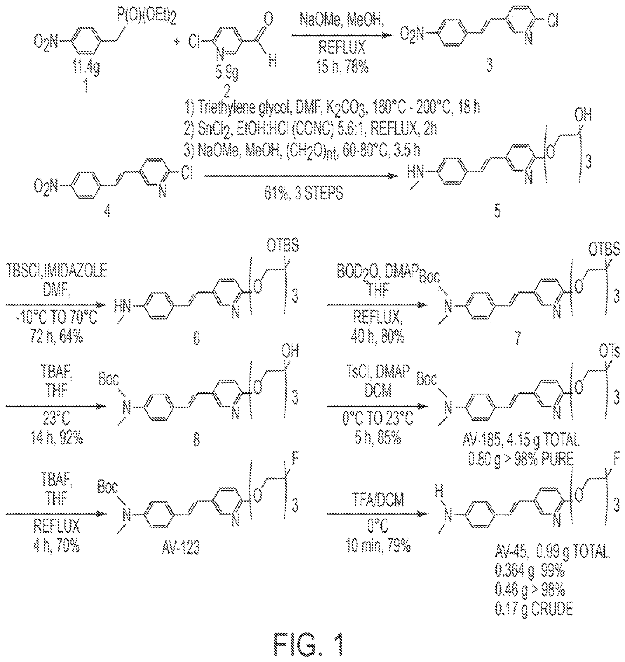

The invention involves synthesis methods for an ¹⁸F-radiolabeled styrylpyridine tosylate precursor, AV-105 (shown in FIG. 1). The AV-105 synthesis method utilizes a primary tosylate instead of a primary mesylate as an active leaving group. Use of the tosylate precursor for radiofluorination is beneficial, at least for the reason that the tosylate generated during the radio fluorination reaction is readily removed by chromatographic means from the ¹⁸F-radiolabeled imaging product. FIG. 1 depicts an initial synthesis of AV-105 and a synthesis of cold AV-45. Use of p-toluenesulfonate (tosylate) as an active leading group eliminates the need for methansulfonate (mesylate), which is not preferred due to genotoxic properties of methane sulfonic acid, a side product formed when mesylate is utilized. Further, p-toluenesulfonic acid is more readily detected than methane sulfonic acid and therefore, can be purified more easily using liquid chromatography with UV detection. As further shown in FIG. 1, 4-dimethylaminopyridine (DMAP) is included in the Boc protection step (6 to 7) and the tosylation step (8) to produce AV-105. The inclusion of DMAP in the tosylation step results in a reduction in the amount of time required for complete conversion of the starting material. The presence of DMAP also inhibits decomposition upon extended reaction times. This synthetic is capable of producing small (e.g., gram) quantities of AV-105, however it was not readily scalable for the production of 50 gram or larger quantities of AV-105.

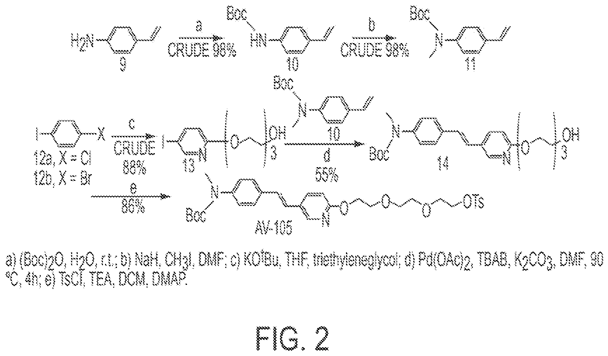

An alternative synthesis method for AV-105 is illustrated in FIG. 2. This convergent synthesis method uses p-toluenesulfonyl chloride rather than the typical methanesulfonyl chloride to generate a pseudo-halide. The use of the Heck reaction as shown in FIG. 2 increases the convergency of synthesis, thereby making the synthesis more efficient and leading to higher yields. However, the yields reported in FIGS. 1 and 2 are for illustrative purposes only and do not represent maximum obtainable yields. As can be appreciated by one skilled in the art, the synthetic methods for producing AV-105 described herein may be further optimized by use of purification techniques applied to the intermediate reactants or the AV- 105 itself. Such techniques may include, for example, recrystallization, solvent-solvent extraction or column chromatographic separation methods capable of removing small amounts of reagent impurities or reaction side-products. Nevertheless, the methods described herein are capable of producing AV-105 in 40% or greater overall yield with a purity of greater than about 95%.

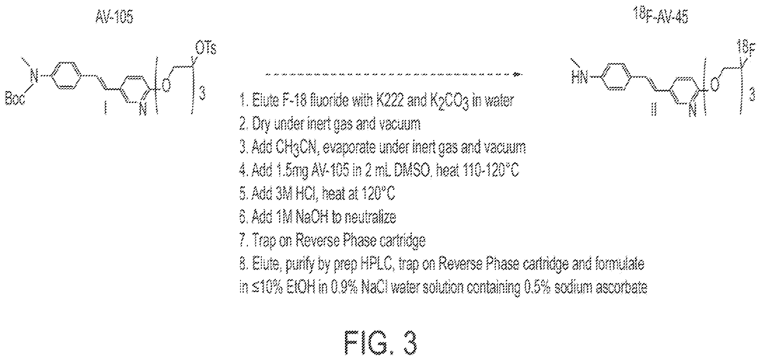

The radiosynthesis according to the invention using a primary alkyl tosylate to produce β-amyloid binding radiopharmaceutical ¹⁸F-AV-45 is shown in FIG. 3. Following the radio labeling process of aspects of the invention, ¹⁸F-AV-45 (labeled as Formula II in FIG. 3) is formulated in a saline solution. The saline solution comprises 10% (v/v) of ethyl alcohol and 0.5 % (w/v) sodium ascorbate and has a pH between 4.5 and 8.0. Without wishing to be bound by theory, the presence of sodium ascorbate minimizes radio lysis during the purification of crude ¹⁸F-A V-45 and in the final product diluent to increase stability and shelf life. In one embodiment of the invention, the nominal concentration of sodium ascorbate is 0.5% (w/v).

There is also disclosed a method for detecting a neurodegenerative disease in a patient including administering a radiopharmaceutical composition capable of binding to a target associated with a neurodegenerative disease comprising an effective amount of an ¹⁸F-radiolabeled amyloid imaging compound, such as ¹⁸F-AV-45, or an alternative F-18 labeled amyloid binding radiopharmaceutical, with 10% (v/v) of ethyl alcohol, and 0.5% (w/v) of sodium ascorbate to the patient; imaging a portion of the patient comprising a region of the patient wherein the target is expected to be positioned; and detecting the target. The region of the patient may include at least a portion of the brain.

The radiopharmaceutical composition can be used in the diagnosis of neurodegenerative disease such as, for example, dementia, cognitive impairment, Alzheimer's Disease (AD), Parkinson's Disease (PD), Dementia with Lewy Bodies (DLB), Vascular Dementia (VaD), and combinations thereof

The ¹⁸F-radiolabeled pharmaceutical composition may be administered by any method known in the art. For example, in a particular aspect, the radiopharmaceutical composition may be administrated by injection. In particular, methods of administration may include, but are not limited to, intravascular injection, intravenous injection, intraperitoneal injection, subcutaneous injection, and intramuscular injection. The ¹⁸F-radiolabeIed pharmaceutical composition may also be administered in unit dosage form, e.g., as an intravenous administration. In some embodiments, the radiopharmaceutical composition may be prepared in a unit dose syringe containing an appropriate quantity of the radiopharmaceutical.

The ¹⁸F-radiolabeled pharmaceuticals may be imaged using any suitable technique known in the art including Positron Emission Tomography (PET). Single Photon Emission Computed Tomography (SPECT), PET with concurrent computed tomography imaging (PET/CT), PET with concurrent magnetic resonance imaging (PET/MRI) or a combination thereof.

The ¹⁸F-radiopharmaceutical compositions possess both high radiochemical purity and high radiosynthetic yield. The shelf-lives of the ⁱ⁸F-radiolabeled pharmaceuticals may vary and may depend upon various aspects of the procedure. Generally, the shelf-lives of the ¹⁸F-radiolabeled pharmaceuticals are greater 8 hours after production when formulated in the compositions described herein. There is also disclosed a formulation of the ¹⁸F-labeled compound that is both safe for administration to humans and stable to radio lysis or other chemical degradation over the time period (e.g., up to 8-10 hours) of shipment to hospitals or other imaging centers. For example, in one embodiment, an ¹⁸F-AV-45 radiopharmaceutical composition comprising ethyl alcohol and sodium ascorbaie maintains stability (greater than 90% RCP) for up to 20 hours post-production

### EXAMPLES

In order that the invention disclosed herein may be more efficiently understood, the following examples are provided. These examples are for illustrative purposes.

### Example 1.0. Synthesis of AV-105 Tosylate Precursor to ¹⁸F-AV-45.

The synthetic route for the scale-up synthesis of tosylate precursor (E)-2-(2-(2-(5-(4-(tert-butoxycarbonyl(melhyl)amino)styryl)pyridin-2-yloxy)ethoxy)ethoxy)ethyl 4-methylbenzenesulfonate ("AV-105") of ((E)-4-(2-(6-(2-(2-(2-[¹⁸F]fluoroethoxy)ethoxy)ethoxy) pyridin-3-yl)vinyl)-N-methylbenzenamine) ("¹⁸F-AV-45") in accordance with one embodiment of the invention is shown in FIG. 2. Mono-Boc-protected vinylaniline **10** was prepared by vigorously stirring vinylaniline with di-tert-butyl dicarbonate in water at room temperature for 2 hours. Mono-Boc-protected vinylaniline **10** was precipitated and filtered to provide a 98% yield, which was used without further purification. Methylation of the intermediate using sodium hydride and methyl iodide in dimethylformamide (DMF) gave crude product tert-Butyl methyl(4-vinylphenyl)carbamate **11** (88% yield), which was also used without further purification. 2-Bromo-5-iodopyridine was alkylated with triethylene glycol using potassium tert-butoxide. At least 4 eq of triethylene glycol to iodopyridine were used to ensure the mono-alkylated product 2-(2-(2-(5-Iodopyridin-2-yloxy)ethoxy)ethoxy)ethanol **13** was the major product. The mono-alkylated product **13** (88% yield) was not purified and was used directly in reaction step d. A Heck reaction between the crude product **11** and the mono-alkylated product **13** was conducted using palladium acetate, tetrabutylammonium bromide, and potassium carbonate in DMF to give the styrylpyridine **14,** which was purified by Biotage medium pressure flash chromatography (55% yield). Tosylation of the styrylpyridine **14** was performed using tosyl chloride, triethylamine, and DMAP in DCM to obtain AV-105, which was purified using Biotage medium pressure flash chromatography. The overall yield starting from 2-bromo-5-iodopyridine was 40% (3 steps).

### Example 1.1. tert-Butyl 4-vinylphenylcarbamate.

Vinylaniline (3.75g, 31.4 mmol) and di-tert-butyl dicarbonate (7.55g, 34.6 mmol) were stirred vigorously in water (23 mL) at room temperature for two hours. Precipitates were filtered, and the remaining filter cake was redissolved in ethyl acetate (50 mL). The organic layer was washed with water (50 mL) and brine (50 mL), dried over anhydrous sodium sulfate, filtered, and concentrated under reduced pressure to obtain crude Mono-Boc-protected vinylaniline 5 (6.75 g, 98%) as a pinkish solid, which may be used in the next step without further purification.

### Example 1.2. tert-Butyl methyl(4-vinylphenyl)carbamate.

Under a nitrogen atmosphere, sodium hydride (1.11g 46.2 mmol) was added into anhydrous DMF (80 mL), and the suspension was cooled to 0 °C using an ice bath. Mono-Boc-protected vinylaniline **10** (6.75g, 30.8 mmol) dissolved in anhydrous DMF (30 mL) was added within 30 minutes through an additional funnel. The reaction mixture was allowed to warm to room temperature, and methyl iodide (8.75 g, 61.6 mmol) was added within 30 minutes using a syringe. After stirring at room temperature for another 1.5 hours, the mixture was poured onto ice (200 g) and extracted with ethyl acetate (200 mL). The organic layer was separated from the aqueous layer, washed with water (100 mL) and brine (50 mL), dried over anhydrous sodium sulfate, filtered, and concentrated under reduced pressure to obtain crude product **11** (6.3 g, 88%) as a reddish oil.

### Example 1.3. 2-(2-(2-(5-Iodopyridin-2-yloxy)ethoxy)ethoxy)ethanol.

Potassium tert-butoxide (869 mg, 7.7 mmol) was added in portions to a solution of 2-bromo-5-iodopyridine (2g, 7.04 mmol) and methylene glycol (4.23g, 28.1 mmol) in tetrahydrofuran (THF) (40 mL). The reaction mixture was then refluxed for 20 h. The reaction mixture was concentrated under reduced pressure to remove the THF solvent. The concentrate was diluted with water (100 mL) and extracted with ethyl acetate (50 mL x 2). The combined organic layers were washed with water (50 mL) then brine (50 mL), dried over anhydrous sodium sulfate, filtered, and concentrated under reduced pressure to obtain the mono-alkylated product 2-(2-(2-(5-Iodopyridin-2-yloxy)ethoxy)ethoxy)ethanol **13** (2.2 g, 88%) as a light yellowish oil that solidified after sitting overnight at ambient temperature.

### Example 1.4. (E)-tert-Butyl 4-(2-(6-(2-(2-(2-hydroxyethoxy)ethoxy)ethoxy)pyridin-3-yl)vinyl)phenyl(methyl)carbamate.

The coupling partners tert-Butyl methyl(4-vinylphenyl)carbamate **11** (1.29 g, 5.55 mmol) and 2-(2-(2-(5-Iodopyridin-2-yloxy)ethoxy)elhoxy)ethanol **13** (1.96 g, 5.55 mmol), together with palladium acetate (62.3 mg, 0.278 mmol), tetrabutylammonium bromide (5.53 g, 16.65 mmol), and potassium carbonate (3.83 g, 27.75 mmol) were added to anhydrous DMF (80 mL). The reaction mixture was degassed with N₂ for 5 minutes and heated at 100 °C overnight. The volume of the reaction was reduced 50% under vacuum, and the mixture was partitioned between ethyl acetate (200 mL) and water (400 mL). The aqueous layer was separated and extracted with additional ethyl acetate (150 mL). The organic layers were combined, washed with water (100 mL) and brine (100 mL), dried over anhydrous sodium sulfate, and concentrated under reduced pressure. The residue was purified by Biotage medium pressure flash column chromatography (45% ethyl acetate in hexanes; 65% ethyl acetate in hexanes) to give (E)-tert-Butyl 4-(2-(6-(2-(2-(2-hydroxyethoxy)ethoxy)ethoxy)pyridin-3-yl)vinyl)phenyl(methyl) carbamate **14** (1.4 g, 55 %).

### Example 1.5. (E)-2-(2-(2-(5-(4-(tert-butoxycarbonyl(raethyl)amino)styryl)pyridin-2-yloxy)ethoxy)ethoxy)ethyl 4-methylbenzenesulfonate (AV-105).

The (E)-tert-Butyl 4-(2-(6-(2-(2-(2-hydroxyethoxy)ethoxy)ethoxy)pyridin-3-yl)vinyl)phenyl(methyl)carbamate **14** (1.3g, 2.83 mmol) was dissolved in DCM (50 mL) followed by the addition of toluenesulfonyl chloride (1.08 g, 5.68 mmol), triethylamine (0.86 g, 8.49 mmol), and DMAP (24 mg, 0.2 mmol). The mixture was stirred at room temperature for 6 hours. Water (50 mL) was added. The organic layer was separated, washed with brine, dried over anhydrous sodium sulfate, concentrated under reduced pressure, and purified by Biotage medium pressure flash column chromatography (start from 30% ethyl acetate in hexane and way up to 40% ethyl acetate in hexane) to obtain compound AV-105 (1.5 g, 86%) as a clear, light yellow oil, which eventually solidified after a few days under vacuum.

### Example 2.0. Radiosynthesis of ¹⁸F-AV-45 from AV-105.

[¹⁸F]fluoride activity was trapped on an anion exchange cartridge and eluted to the reaction vessel using aqueous K.2.2.2-K₂CO₃ solution (potassium carbonate and Kryptofix) and CH₃CN. The eluted activity was dried under vacuum. The [¹⁸F]fluoride was further dried by addition of CH₃CN to the reactor vessel, and the water-CH₃CN azeotrope was evaporated with heating. AV-105 (1.5 mg in 2.0 mL of DMSO) was added to the reactor vessel. The vessel mixture was heated to 110-120 °C for the ¹⁸F-radiolabeling reaction. A solution of 3M HCl was added to the vessel, and the resulting mixture was heated to 120 °C for 5 minutes to remove the N-Boc-group. After cooling, a solution of 1M NaOH solution was added for neutralisation. The solution was loaded onto a reverse phase cartridge. The isolated crude ¹⁸F-AV-45 on the cartridge was washed with about 4 mL of water for injection (WFI) containing 5% w/v sodium ascorbate. The ¹⁸F-AV-45 was then eluted using 1.5 mL of CH₃CN into a reservoir containing 2 mL of WFI containing 5% w/v sodium ascorbate and 1 mL of high performance liquid chromatography (HPLC) solvent. The ¹⁸F-AV-45 was then loaded onto a semi-preparative HPLC column and subjected to purification. The HPLC fraction containing the purified ¹⁸F-AV-45 was collected in a reservoir containing 20 mL of WFI containing 0.5% w/v sodium ascorbate. The diluted solution was passed through a SEP-PAK ® C-18 cartridge and the trapped ¹⁸F-AV-45 was washed with 15 mL of WF1 containing 0.5% w/v sodium ascorbate. The ¹⁸F-AV-45 was eluted off the SEP-PAK® Light C-18 cartridge using 1 mL of EtOH (USP) into 9 mL of 0.9% Sodium Chloride Injection (sterile, USP) containing 0.5% w/v sodium ascorbate (USP). This solution was then dispensed through a 0.22 µm sterilizing filter into a 10 or 30 mL sterile pre-crimped septum-sealed container. The ¹⁸F-AV-45 drug product may be diluted with Sodium Chloride Injection (0.9%, USP, sterile) containing NMT 10% v/v EtOH (USP) and 0.5% w/v sodium ascorbate (USP).

### Example 3.0. Formulation for Solubilization of ¹⁹F-AV-45 and Stabilization of ¹⁸F-AV-45.

The 18F-AV-45 radiopharmaceutical formulation/composition was evaluated for the solubility of the non-radioactive ¹⁹F-AV-45, which is also formed in quantities of 1-5 µg/mL during the radiosynthesis of ¹⁸F-AV-45 due to the presence of small quantities of ¹⁹F-fluoride from tubing, valves and other sources in the radiosynthesis system. In the absence of ethanol in the composition, particulate matter may be observed due to the low solubility of ¹⁹F-AV -45. The addition of 10% ethanol to the radiopharmaceutical composition was, therefore, evaluated to determine if the solubility of ¹⁹F-A V-45 was sufficient to avoid the potential of precipitate formation. The solubility limit of AV-45 in the drug product composition (10% (v/v) EtOH and 0.5% (w/v) sodium ascorbate in 0.9% aqueous sodium chloride) was determined to be 17 µg/mL at ambient temperature. A visible precipitate was noticeable in solutions with concentrations ≥ 22 µg/mL. Samples prepared at concentrations ≤ 20 µg/mL were evaluated by HPLC analysis following centrifugation. Samples with≤ 20 µg/mL were also tested after storage for one week at ambient temperature. There was no change in concentration after one week of storage at ambient temperature.

The ¹⁸F-AV-45 radiopharmaceutical compositions of embodiments of the invention are remarkably stable to radio lysis or chemical degradation. Sodium ascorbate is used to minimize radio lysis during the purification of ¹⁸F-AV-45 and in the final drug product solution to increase stability and shelf life. Ethanol at 1-10% (v/v) in aqueous solution aids in the solubilization of ¹⁸F-AV-45. To demonstrate the stabilization of ¹⁸F-AV-45 in the final drug product, research compositions were manufactured with and without sodium ascorbate. The compositions were analyzed at end-of-synthesis (EOS) using reverse HPLC, equipped with both an ultra violet (UV) detector and radiochemical detector. The radiochemical purity of ¹⁸F-AV-45 was monitored by HPLC with radiometric detection and the purity of ¹⁹F-AV-45 was monitored by HPLC UV detection in the drug product by Area Percent Normalization. The area percent values for both radiochemical and ultraviolet analysis are shown in Tables 1 and 2.

At EOS, the radiochemical purity of the ¹⁸F-AV-45 composition prepared without sodium ascorbate was 84% and decomposed further such that at 2 hours the purity was 80%. The purity of the ¹⁸F-AV-45 composition manufactured with sodium ascorbate was significantly higher. At EOS, the purity was 96% with negligible decomposition at 2 hours post EOS (purity of 95%).

**Table 1. Percent Radiochemical Purity Analysis of ¹⁸F-AV-45 Injection at End of Synthesis (EOS)**

| **Radioactive Analytes** | **AV4520080304** | | **AV4520080603** | |
|---|---|---|---|---|
| | Manufactured With Out Sodium Ascorbate (444 MBq/mL) | | Manufactured With Sodium Ascorbate (1036 MBq/mL) | |
| Time | EOS | 2-hour Post EOS | EOS | 2-hour Post EOS |
| % ¹⁸F AV-45 Purity | 84% | 80% | 96% | 95% |
| Unknown 1 (RRT =0.25) | ND | ND | 2% | 3% |
| Unknown 2 (RRT =0.31) | 2% | 4% | ND | ND |
| Unknown 3 (RRT =0.36) | ND | ND | 2% | 2% |
| Unknown 4 (RRT =0.40) | 14% | 16% | ND | ND |

Sodium ascorbate had a similar stabilizing affect on ¹⁹F-AV-45 and minimized the formation of non-radioactive impurities detected by UV, as shown in Table 2

**Table 2. UV Purity Analysis of ¹⁹F-AV-45 in ¹⁸F-AV-45 Injection by Area Percent at End of Synthesis (EOS) and 2 Hours Post EOS***

| | **AV4520080304** | | **AV4520080603** | |
|---|---|---|---|---|
| **UV Analytes A5 350nm** | Manufactured With Out Sodium Ascorbate (444 MBq/mL) | | Manufactured With Sodium Ascorbate (1036 MBq/mL) | |
| Time | EOS | 2-h Post EOS | EOS | 2-h Post EOS |
| % ¹⁹F-AV-45 (RRT =1.00) | 83% | 76% | 100% | 100% |
| Unknown 1 (RRT =0.37) | 6% | 8% | ND | ND |
| Unknown 2 (RRT =0.36) | 1% | 1% | ND | ND |
| Unknown 3 (RRT =0.81) | 4% | 4% | ND | ND |
| Unknown 4 (RRT =0.92) | | 1% | ND | ND |
| Unknown 5 (RRT =0.95) | 5% | 5% | ND | ND |
| Unknown 6 (RRT= 1.11) | ND | 2% | ND | ND |

| | | | | |
|---|---|---|---|---|
| *Only impurities greater than or equal to 1% are included in this table | | | | |

Table 3 shows extended stability of the ¹⁸F-AV-45 radiopharmaceutical in approximately 10% (v/v) of ethanol in normal saline containing 0.5% (w/v) of sodium ascorbate. As noted, the radiochemical purity of the ¹⁸F compound is maintained for up to approximately 20 hours after production.

**Table 3. Extended Stability of ¹⁸F-AV-45 Injection Manufactured and Formulated With Sodium Ascorbate at 0.5%**

| **Lot Number** | **Storage Conditions** | **% RCP At EOS** | **% RCP (Post EOS)** |
|---|---|---|---|
| AV4520071217_1 | Ambient Temperature | 99% | 99% (21 hours) |
| AV4520071220_1 | Ambient Temperature | 99% | 100% (17 hours) |
| AV4520080114_1 | Ambient Temperature | 100% | 100% (20 hours) |

An additional study was performed to demonstrate the stability of the ¹⁸F-AV-45 radiopharmaceutical in 10% (v/v) of ethanol in normal saline containing 0.5% (w/v) of sodium ascorbate when stored in a FLUROTEC®. The stability of the drug product was evaluated on three separate manufacturing lots of ¹⁸F-AV-45. Vials were stored upright and inverted at ambient and 40 °C temperatures, and upright at 50 °C. Drug product was sampled at the specified intervals over a 12 hour testing period and evaluated for radiochemical purity and strength. The constant RCP over 12 hours and < 5% drift of the drug product strength at all temperatures tested demonstrates the stability of the drug substance in the drug product as well as the minimal interaction of the drug substance with the vial stopper.

## Claims

1. A method for producing a radiopharmaceutical composition for positron emission tomography (PET) imaging of neurodegenerative diseases of the brain comprising:
an effective amount of ((E)-4-(2-(6-(2-(2-(2-[¹⁸F] fluoroethoxy)ethoxy)ethoxy)pyridin-3-yl)vinyl)-N-methylbenzenamine);
10.0% (v/v) of ethyl alcohol; and
0.5 % (w/v) of sodium ascorbate,
in 0.9% (w/v) aqueous sodium chloride,
wherein the method comprises the steps of:
preparing a mono Boc-protected vinylaniline compound;
converting the vinylaniline compound to a methyl, t-butyl carbamate derivative;
reacting 2-halo 5-iodopyridine with triethyleneglycol;
reacting the methyl, t-butyl carbamate derivative with the resultant compound of the step of reacting 2-halo 5-iodopyridine with triethyleneglycol to produce (E)-tert-Butyl 4-(2-(6-(2-(2-(2-hydroxyethoxy)ethoxy)ethoxy)pyridin-3-yl)vinyl)phenyl(methyl)carbamate; and
reacting the (E)-tert-Butyl 4-(2-(6-(2-(2-(2-hydroxyethoxy)ethoxy)ethoxy)pyridin-3-yl)vinyl)phenyl(methyl)carbamate with tosyl chloride to form (E)-2-(2-(2-(5-(4-(tert-butoxycarbonyl(methyl)amino)styryl)pyridin-2-yloxy)ethoxy)ethoxy)ethyl 4-methylbenzene sulfonate;
reacting (E)-2-(2-(2-(5-(4-(tert-butoxycarbonyl(methyl)amino)styryl)pyridin-2-yloxy)ethoxy)ethoxy)ethyl
4-methylbenzene sulfonate and an [18F]-fluoride ion in dimethylsulfoxide (DMSO) solution or high-boiling point aprotic solvent to produce (*E*)-4-(2-(6-(2-(2-(2-[¹⁸F]fluoroethoxy)ethoxy)ethoxy)pyridin-3-yl)vinyl)-N-methylbenzenamine);
isolating the (*E*)-4-(2-(6-(2-(2-(2-[¹⁸F]fluoroethoxy)ethoxy)ethoxy)pyridin-3-yl)vinyl)-N-methylbenzenamine);
purifying the (*E*)-4-(2-(6-(2-(2-(2-[¹⁸F]fluoroethoxy)ethoxy)ethoxy)pyridin-3-yl)vinyl)-N-methylbenzenamine); and
formulating the (*E*)-4-(2-(6-(2-(2-(2-[¹⁸F]fluoroethoxy)ethoxy)ethoxy)pyridin-3-yl)vinyl)-N-methylbenzenamme) in an 0.9% (w/v) aqueous sodium chloride-ethyl alcohol solution comprising ethyl alcohol at 10.0% (v/v) of the total composition and sodium ascorbate at 0.5 % (w/v) of the total composition.

2. A compound of the formula

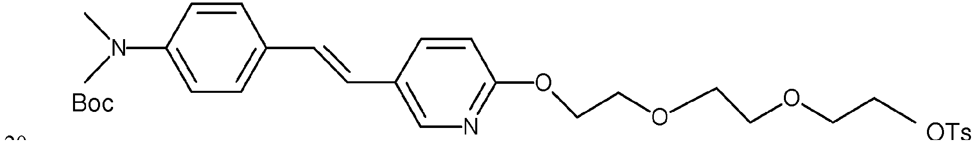

## Patentansprüche

1. Verfahren zur Herstellung einer radiopharmazeutischen Zusammensetzung für die bildliche Darstellung neurodegenerativer Krankheiten des Gehirns mittels Positronen-Emissions-Tomographie, wobei diese Folgendes umfasst:
eine wirksame Menge an ((E)-4-(2-(6-(2-(2-(2-[¹⁸F]-fluorethoxy)ethoxy)ethoxy)pyridin-3-yl)vinyl)-N-methylbenzolamin);
10,0 % (v/v) an Ethylalkohol; und
0,5 % (w/v) an Natriumascorbat,
in 0,9 %igem (w/v) wässrigem Natriumchlorid,
wobei das Verfahren die folgenden Schritte umfasst:
Herstellen einer mono-Boc-geschützten Vinylanilinverbindung,
Umwandeln der Vinylanilinverbindung in ein Derivat von Methyl-, t-Butyl-Carbamat;
Umsetzen von 2-Halogen-5-lodpyridin mit Triethylenglykol;
Umsetzen des Derivats von Methyl-, t-Butyl-Carbamat mit der Verbindung, welche im Schritt des Umsetzens von 2-Halogen-5-lodpyridin mit Triethylenglykol erhalten wird, um (E)-tert.-Butyl-4-(2-(6-(2-(2-(2-hydroxy-ethoxy)ethoxy)ethoxy)pyridin-3-yl)vinyl)phenyl(methyl)carbamat herzustellen; und
Umsetzen des (E)-tert.-Butyl-4-(2-(6-(2-(2-(2-hydroxyethoxy)ethoxy)ethoxy)-pyridin-3-yl)vinyl)phenyl(methyl)carbamats mit Tosylchlorid, um (E)-2-(2-(2-(5-(4-(tert.-Butoxycarbonyl(methyl)amino)styryl)pyridin-2-yloxy)ethoxy)ethoxy)-ethyl-4-methylbenzolsulfonat zu erhalten;
Umsetzen des (E)-2-(2-(2-(5-(4-(tert.-Butoxycarbonyl(methyl)amino)styryl)-pyridin-2-yloxy)ethoxy)ethoxy)ethyl-4-methylbenzolsulfonats und des [18F]-Fluorid-Ions in einer Dimethylsulfoxid(DMSO)-Lösung oder einem hochsiedenden aprotischen Lösungsmittel, um (*E*)-4-(2-(6-(2-(2-(2-[¹⁸F]-Fluorethoxy)ethoxy)ethoxy)pyridin-3-yl)vinyl)-N-methylbenzolamin) herzustellen;
Isolieren des (*E*)-4-(2-(6-(2-(2-(2-[¹⁸F]-Fluorethoxy)ethoxy)ethoxy)pyridin-3-yl)-vinyl)-N-methylbenzolamins);
Aufreinigen des (*E*)-4-(2-(6-(2-(2-(2-[¹⁸F]-Fluorethoxy)ethoxy)ethoxy)pyridin-3-yl)vinyl)-N-methylbenzolamins); und
Formulieren des (*E*)-4-(2-(6-(2-(2-(2-[¹⁸F]-Fluorethoxy)ethoxy)ethoxy)pyridin-3-yl)vinyl)-N-methylbenzolamins) in einer 0,9 %igen (w/v) wässrigen Lösung von Natriumchlorid-Ethylalkohol, welche 10,0 % (v/v) an Ethylalkokol, bezogen auf die Zusammensetzung insgesamt, und 0,5 % (w/v) an Natriumascorbat, bezogen auf die Zusammensetzung insgesamt, umfasst.

2. Verdindung nach der Formel

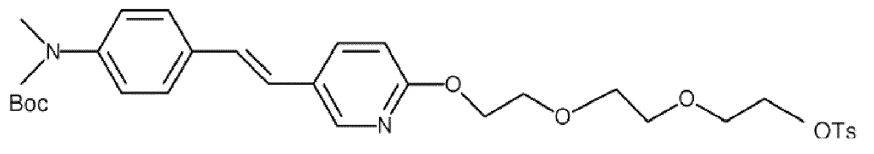

## Revendications

1. Procédé de production d'une composition radiopharmaceutique pour imagerie par tomographie par émission de positrons (TEP) de maladies neuro-dégénératives du cerveau, comprenant :
une quantité efficace de ((E)-4-(2-(6-(2-(2-(2-[¹⁸F]fluoroéthoxy)éthoxy)éthoxy)pyridin-3-yl)vinyl)-N-méthylbenzènamine) ;
10,0 % (v/v) d'alcool éthylique ; et
0,5 % (p/v) d'ascorbate de sodium,
dans une solution aqueuse de chlorure de sodium à 0,9 % (p/v),
dans lequel le procédé comprend les étapes de :
préparation d'un composé vinylaniline protégé par un mono Boc ;
conversion du composé vinylaniline en un dérivé t-butyl carbamate de méthyle ;
réaction de 2-halogéno-5-iodopyridine avec du triéthylèneglycol ;
réaction du dérivé t-butyl carbamate de méthyle avec le composé résultant de l'étape de réaction de la 2-halogéno-5-iodopyridine avec le triéthylèneglycol pour produire du 4-(2-(6-(2-(2-(2-hydroxyéthoxy)éthoxy)éthoxy)pyridin-3-yl)vinyl)phényl(méthyl)carbamate de (E)-tert-butyle ; et
réaction du 4-(2-(6-(2-(2-(2-hydroxyéthoxy)éthoxy)éthoxy)pyridin-3-yl)vinyl)phényl(méthyl)carbamate de (E)-tert-butyle avec du chlorure de tosyle pour former du 4-méthylbenzène sulfonate de (E)-2-(2-(2-(5-(4-(tert-butoxycarbonyl(méthyl)amino)styryl)pyridin-2-yloxy)éthoxy)éthoxy)éthyle ;
réaction du 4-méthylbenzène sulfonate de (E)-2-(2-(2-(5-(4-(tert-butoxycarbonyl(méthyl)amino)styryl)pyridin-2-yloxy)éthoxy)éthoxy)éthyle et d'un ion [¹⁸F]-fluorure dans une solution de diméthylsulfoxyde (DMSO) ou un solvant aprotique à point d'ébullition élevé pour produire de la (E)-4-(2-(6-(2-(2-(2-[¹⁸F]fluoroéthoxy)éthoxy)éthoxy)pyridin-3-yl)vinyl)-N-méthylbenzènamine) ;
l'isolement de la (*E*)-4-(2-(6-(2-(2-(2-[¹⁸F]fluoroéthoxy)éthoxy)éthoxy)pyridin-3-yl)vinyl)-N-méthylbenzènamine) ;
la purification de la (*E*)-4-(2-(6-(2-(2-(2-[¹⁸F]fluoroéthoxy)éthoxy)éthoxy)pyridin-3-yl)vinyl)-N-méthylbenzènamine) ; et
la formulation de la (*E*)-4-(2-(6-(2-(2-(2-[¹⁸F]fluoroéthoxy)éthoxy)éthoxy)pyridin-3-yl)vinyl)-N-méthylbenzènamine) dans une solution aqueuse à 0,9 % (p/v) de chlorure de sodium-alcool éthylique comprenant de l'alcool éthylique à 10,0 % (v/v) de la composition totale et de l'ascorbate de sodium à 0,5 % (p/v) de la composition totale.

2. Composé de formule
